# EUROPEAN PATENT APPLICATION

(11) **EP 3 660 853 A2**
(43) Date of publication of application: **03.06.2020**
(21) Application number: 19201774.7
(22) Date of filing: 07.10.2019
(51) Int. Cl.: G16B 40/10, G16B 40/20

(54) **MASS SPECTROMETER, MASS SPECTROMETRY METHOD, AND MASS SPECTROMETRY PROGRAM FOR DISCRIMINATING A STRAIN IN A MICROORGANISM**

(30) Priority: 10.10.2018 JP 2018191764
(71) Applicant: Shimadzu Corporation, Kyoto 604-8511 (JP)
(72) Inventor: YAMADA, Yoshihiro, Nakagyo-ku, Kyoto 604-8511 (JP); FUKUYAMA, Yuko, Nakagyo-ku, Kyoto 604-8511 (JP); TAMURA, Hiroto, Nagoya, Aichi 468-8502 (JP)
(74) Representative: Mewburn Ellis LLP

(57) **Abstract**

Each of a plurality of mass spectral data that includes a microorganism of which strain is known is acquired as training data by a training data acquirer. A sample corresponding to each training data includes an additive, and a matrix is mixed with the sample. A discrimination analysis model for discriminating a strain based on the acquired plurality of training data is produced by a model producer by performing machine learning. Mass spectral data that includes a microorganism of which strain is unknown is acquired as target data by a target data acquirer. A sample corresponding to the target data includes the additive, and the matrix is mixed with the sample. A strain of the microorganism corresponding to the acquired target data is discriminated by a discriminator based on the produced discrimination analysis model for each strain and the acquired target data.

## Description

### BACKGROUND OF THE INVENTION

### Field of the Invention

The present invention relates to a mass spectrometer that identifies or discriminates a microorganism, a mass spectrometry method for identifying or discriminating a microorganism, and a mass spectrometry program for identifying or discriminating a microorganism.

### Description of Related Art

A mass spectrometer is used to identify or discriminate samples of various microorganisms. It is possible to detect a marker peak for identifying or discriminating each sample by comparing a plurality of mass spectra obtained with respect to a plurality of samples. A microorganism identification/discrimination system (hereinafter referred to as the MALDI-MS system) using MALDI-MS (Matrix-assisted Laser Desorption Ionization Mass Spectrometry) is excellent in rapidity and cost performance, and has been rapidly widely used in clinical sites in recent years.

At this time, in the clinical sites, microorganism identification/discrimination using the MALDI-MS system remains at a species level of identification/discrimination. On the other hand, in academic research, it has been reported that a microorganism has been identified or discriminated at a strain level. For example, an article by Yudai Hotta et al., "Classification of the Genus Bacillus Based on MALDI-TOF MS Analysis of Ribosomal Proteins Coded in S10 and spc Operons," Journal of Agricultural and Food Chemistry, 2011, Vol. 59, No. 10, pp. 5222-5230 describes that a theoretical mass of a protein (mainly a ribosomal protein) that is expressed only in a specific strain is calculated based on gene information. Discrimination of a strain is performed depending on whether there is a peak (marker peak) in a mass-to-charge ratio corresponding to the calculated theoretical mass.

### BRIEF SUMMARY OF THE INVENTION

Also in the clinical sites, it is expected that infection routes of microorganisms can be clarified or determination can be made as to whether microorganisms have toxicity by putting the discrimination of strains of microorganisms into practice use. However, it is not easy to discriminate the strains of microorganisms with high accuracy.

An object of the present invention is to provide a mass spectrometer, a mass spectrometry method, and a mass spectrometry program, for enabling higher accuracy in discrimination of the strains of the microorganisms.

The inventors of the present invention have considered producing a discrimination analysis model for discriminating the strains of the microorganisms by performing machine learning using a plurality of mass spectra. As a result of various experiments and considerations, the inventors have found that it is possible to produce a discrimination analysis model that is available for the discrimination of strains by reducing variations in peak intensity of each mass spectrum. Based on this finding, the inventors have conceived of the present invention as described below.
(1) A mass spectrometer according to one aspect of the present invention that discriminates a strain of a microorganism includes a training data acquirer that acquires, as training data, each of a plurality of mass spectral data with respect to a plurality of samples, each sample including a microorganism of which strain is known, an additive, and a matrix mixed with the sample, a model producer that produces a discrimination analysis model for discriminating a strain based on the plurality of training data acquired by the training data acquirer by performing machine learning, a target data acquirer that acquires, as target data, mass spectral data with respect to a sample including a microorganism of which strain is unknown, the additive, and the matrix mixed with the sample, and a discriminator that discriminates the strain of the microorganism corresponding to the target data acquired by the target data acquirer based on the discrimination analysis model for each strain produced by the model producer and the acquired target data.
   In this mass spectrometer, each of the plurality of mass spectral data corresponding to the microorganisms, of which strains are known, is acquired as the training data. The sample corresponding to each training data includes the additive and also includes the matrix mixed with the sample. The discrimination analysis model for discriminating a strain based on the acquired plurality of training data is produced by performing the machine learning. Also, the mass spectral data corresponding to the microorganism, of which strain is unknown, is acquired as the target data. The sample corresponding to the target data includes the additive and also includes the matrix mixed with the sample. The strain of the microorganism corresponding to the acquired target data is discriminated based on the produced discrimination analysis model for each strain and the acquired target data.
   With this configuration, variations in peak intensity of each training data are reduced. As such, it is possible to produce the discrimination analysis model available for the discrimination of the strain by performing the machine learning on the acquired plurality of training data. Further, similarly to each training data, variations in peak intensity of the target data are reduced. This makes it possible to discriminate the strain of the microorganism corresponding to the target data based on the produced discrimination analysis model and the target data. As a result, accuracy of the discrimination of the strain of the microorganism is improved.
(2) The additive may include at least one of a compound that inhibits alkali metal-added ion detection and a surfactant. In this case, variations in peak intensity of each of the plurality of training data and the target data can be efficiently reduced.
(3) The additive may include a methylenediphosphonic acid or decyl-β-D-maltopyranoside. In this case, the variations in peak intensity of each of the plurality of training data and the target data can be more efficiently reduced.
(4) The model producer may produce the discrimination analysis model by a support vector machine or a neural network. In this case, the discrimination analysis model for discriminating the strain with high accuracy can easily be produced.
(5) The matrix may include a sinapic acid. In this case, each of the plurality of training data and the target data can easily be acquired. Moreover, the variations in peak intensity of each of the plurality of training data and the target data can be efficiently reduced.
(6) A mass spectrometry method according to another aspect of the present invention for discriminating a strain of a microorganism includes acquiring, as training data, each of a plurality of mass spectral data with respect to a plurality of samples, each sample including a microorganism of which strain is known, an additive, and a matrix mixed with the sample, producing a discrimination analysis model for discriminating a strain based on the acquired plurality of training data by performing machine learning, acquiring, as target data, mass spectral data with respect to a sample including a microorganism of which strain is unknown, the additive, and the matrix mixed with the sample, and discriminating the strain of the microorganism corresponding to the acquired target data based on the produced discrimination analysis model for each strain and the acquired target data.
   With this mass spectrometry method, it is possible to discriminate the strain of the microorganism corresponding to the target data with high accuracy based on the produced discrimination analysis model and the target data. As a result, the accuracy of discrimination of the strain of the microorganism is improved.
(7) The additive may include at least one of a compound that inhibits alkali metal-added ion detection and a surfactant. In this case, variations in peak intensity of each of the plurality of training data and the target data can be efficiently reduced.
(8) The additive may include a methylenediphosphonic acid or decyl-β-D-maltopyranoside. In this case, the variations in peak intensity of each of the plurality of training data and the target data can be more efficiently reduced.
(9) The producing of the discrimination analysis model may include producing the discrimination analysis model by a support vector machine or a neural network. In this case, the discrimination analysis model for discriminating the strain with high accuracy can easily be produced.
(10) The matrix may include a sinapic acid. In this case, each of the plurality of training data and the target data can easily be acquired. Moreover, the variations in peak intensity of each of the plurality of training data and the target data can be efficiently reduced.
(11) A mass spectrometry program according to still another aspect of the present invention for discriminating a strain of a microorganism executable by a processor, wherein the mass spectrometry program causes the processor to execute processes of acquiring, as training data, each of a plurality of mass spectral data with respect to a plurality of samples, each sample including a microorganism of which strain is known, an additive, and a matrix mixed with the sample, producing a discrimination analysis model for discriminating a strain based on the acquired plurality of training data by performing machine learning, acquiring, as target data, mass spectral data with respect to a sample including a microorganism of which strain is unknown, the additive, and the matrix mixed with the sample, and discriminating the strain of the microorganism corresponding to the acquired target data based on the produced discrimination analysis model for each strain and the acquired target data.

With this mass spectrometry program, it is possible to discriminate the strain of the microorganism corresponding to the target data with high accuracy based on the produced discrimination analysis model and the target data. As a result, the accuracy of discrimination of the strain of the microorganism is improved.

Other features, elements, characteristics, and advantages of the present invention will become more apparent from the following description of preferred embodiments of the present invention with reference to the attached drawings.

### BRIEF DESCRIPTION OF THE SEVERAL VIEWS OF THE DRAWING

Fig. 1 is a diagram showing a configuration of a mass spectrometer according to one embodiment of the present invention;
Fig. 2 is a diagram showing a configuration of a strain discriminator;
Figs. 3A and 3B are diagrams for use in explaining a discrimination analysis model produced by the strain discriminator of Fig. 2;
Fig. 4 is a flowchart showing an algorithm of strain discrimination processing performed by a strain discrimination program;
Fig. 5 is a diagram showing a mass spectrum of a salmonella;
Fig. 6 is a diagram showing results of main component analysis on a plurality of samples;
Fig. 7 is a diagram showing results of main component analysis on a plurality of samples;
Fig. 8 is a diagram for use in explaining combinations of training data and target data in holdout validation;
Figs. 9A and 9B are diagrams showing incorrect discrimination rates in an inventive example and a comparative example by holdout validation;
Fig. 10 is a diagram for use in explaining combinations of training data and target data in cross validation; and
Figs. 11A and 11B are diagrams showing average incorrect discrimination rates in each of the inventive example and the comparative example by cross validation.

### DESCRIPTION OF THE PREFERRED EMBODIMENTS

### (1) Configuration of Mass Spectrometer

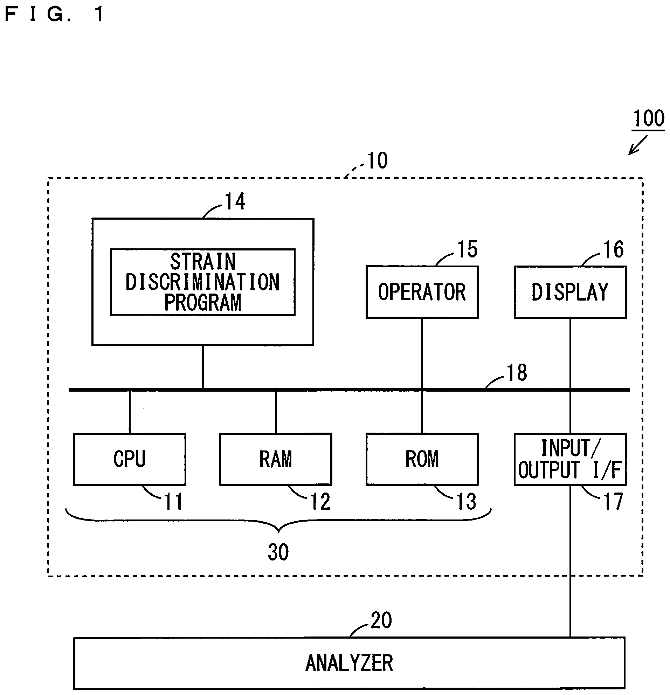

A mass spectrometer, a mass spectrometry method, and a strain discrimination program (mass spectrometry program) according to an embodiment of the present invention will be described in detail below with reference to the drawing. Fig. 1 is a diagram showing a configuration of a mass spectrometer according to one embodiment of the present invention. Fig. 1 mainly shows a configuration of hardware of a mass spectrometer 100. The mass spectrometer 100 includes a processor 10 and an analyzer 20 as shown in Fig. 1.

The processor 10 is constituted by a CPU (Central Processing Unit) 11, a RAM (Radom Access Memory) 12, a ROM (Read Only Memory) 13, a storage device 14, an operator 15, a display 16, and an input/output I/F (interface) 17. The CPU 11, the RAM 12, the ROM 13, the storage device 14, the operator 15, the display 16, and the input/output I/F 17 are connected to a bus 18. The CPU 11, the RAM 12, and the ROM 13 constitute a strain discriminator 30.

The RAM 12 is used as a workspace of the CPU 11. The ROM 13 stores a system program. The storage device 14 includes a storage medium such as a hard disk or a semiconductor memory and stores a strain discrimination program. The CPU 11 executes the strain discrimination program stored in the storage device 14, so that strain discrimination processing is performed as described below.

The operator 15 is an input device such as a keyboard, a mouse or a touch panel. A user can give a predetermined instruction to the analyzer 20 or the strain discriminator 30 by operating the operator 15. The display 16 is a display device such as a liquid crystal display device and displays results of strain discrimination performed by the strain discriminator 30. The input/output I/F 17 is connected to the analyzer 20.

The analyzer 20 produces mass spectral data indicating mass spectra of various samples of microorganisms using MALDI (Matrix-assisted Laser Desorption Ionization). The samples include a sample of which strain is known (hereinafter referred to as training sample) and a sample to be discriminated of which strain is unknown (hereinafter referred to as target sample). A matrix is mixed in each of the training sample and the target sample. Each of the training sample and the target sample includes a predetermined additive.

The matrix includes a sinapic acid, for example. The additive includes at least one of a compound that inhibits detection of alkali metal-added ions and a surfactant. More specifically, the compound inhibiting the detection of the alkali metal-added ions includes a methylenediphosphonic acid (MDPNA). The surfactant includes decyl-β-D-maltopyranoside (DMP). Thus, variations in peak intensity of the produced mass spectral data can be reduced.

The strain discriminator 30 produces a discrimination analysis model based on a plurality of mass spectral data each corresponding to a plurality of the training samples. The strain discriminator 30 discriminates a strain of the target sample based on the produced discrimination analysis model. An operation of the strain discriminator 30 will be described below.

### (2) Strain Discriminator

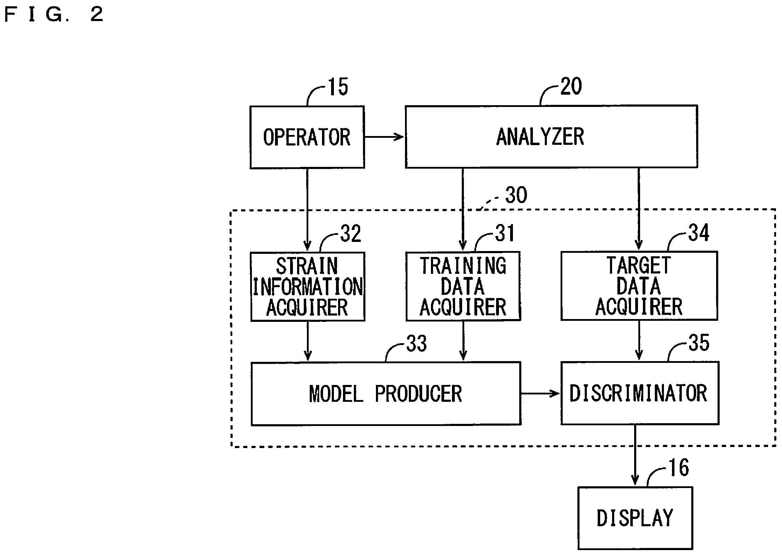

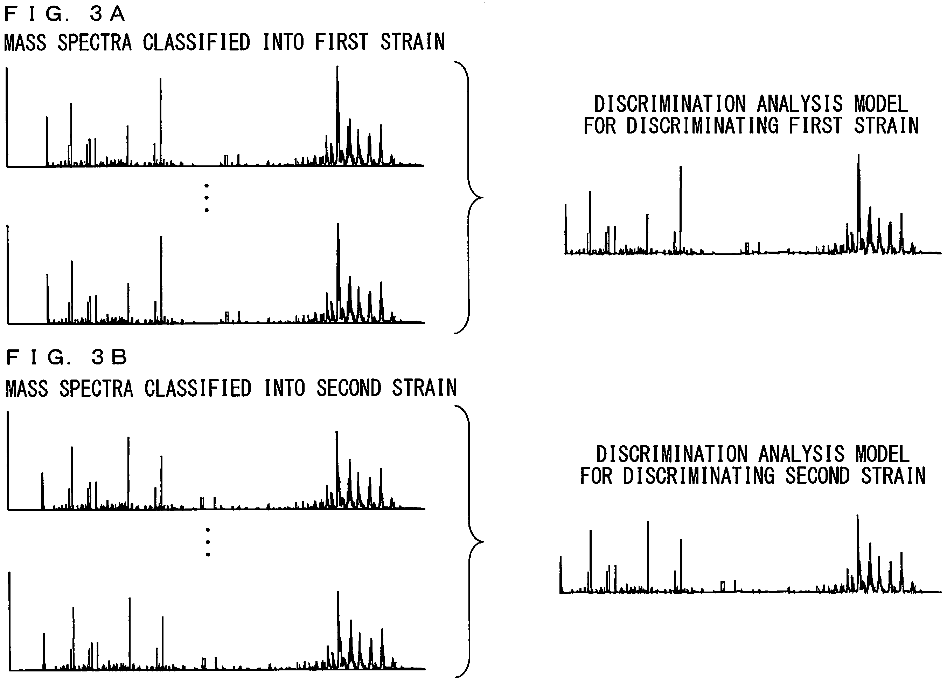

Fig. 2 is a diagram showing a configuration of the strain discriminator 30. Figs. 3A and 3B are diagrams for use in explaining the discrimination analysis model produced by the strain discriminator 30 of Fig. 2. As shown in Fig. 2, the strain discriminator 30 includes, as a function unit, a training data acquirer 31, a strain information acquirer 32, a model producer 33, a target data acquirer 34, and a discriminator 35. The CPU 11 of Fig. 1 executes the strain discrimination program stored in the storage device 14, whereby the function unit of the strain discriminator 30 is implemented. Part or all of the function unit of the strain discriminator 30 may be implemented by hardware such as an electronic circuit.

The training data acquirer 31 acquires a plurality of mass spectral data (hereinafter referred to as training data) each corresponding to the plurality of training samples produced by the analyzer 20. The user can instruct the analyzer 20 to apply a plurality of desired training data to the training data acquirer 31 by operating the operator 15. While the training data acquirer 31 acquires the plurality of training data directly from the analyzer 20 in the example of Fig. 2, the present invention is not limited to this. In the case where the plurality of training data produced by the analyzer 20 are stored in the storage device 14 of Fig. 1, the training data acquirer 31 may acquire the plurality of training data from the storage device 14.

The strain information acquirer 32 acquires from the operator 15 strain information indicating a strain of each of the plurality of training samples corresponding to the plurality of training data acquired by the training data acquirer 31. The user can provide the strain information acquirer 32 with the strain information of each of the plurality of training samples corresponding to the plurality of training data by operating the operator 15.

When training data is produced by the analyzer 20, the user may register strain information corresponding to the training data in the analyzer 20. In this case, each training data and strain information corresponding to the training data can be treated integrally in such a manner that the training data and the corresponding strain information are linked to each other. Thus, when training data is acquired by the training data acquirer 31, strain information corresponding to the training data is automatically acquired from the analyzer 20 or the storage device 14 by the strain information acquirer 32.

The model producer 33 classifies the plurality of training data acquired by the training data acquirer 31 for each strain, based on the strain information acquired by the strain information acquirer 32. Also, the model producer 33 performs machine learning (supervised learning) using the plurality of training data classified into the same strain, thereby to produce, as a discrimination analysis model, a pattern of a mass spectrum for discriminating the strain. The discrimination analysis model is preferably produced by a support vector machine (SVM) or a neural network (NN).

The left column of Fig. 3A shows a plurality of mass spectra based on the plurality of training data classified into a first strain. The right column of Fig. 3A shows a discrimination analysis model for discriminating the first strain produced by performing the machine learning on the plurality of training data shown in the left column of Fig. 3A. The left column of Fig. 3B shows a plurality of mass spectra based on the plurality of training data classified into a second strain. The right column of Fig. 3B shows a discrimination analysis model for discriminating the second strain produced by performing the machine learning on the plurality of training data shown in the left column of Fig. 3B.

While a target of the discrimination analysis models is a sequential waveform in the examples of Figs. 3A and 3B, the present invention is not limited to this. The target of the discrimination analysis models may be a discrete peak list (a set of a peak mass-to-charge ratio and peak intensity). To facilitate understanding, each mass spectrum of Fig. 3A and each mass spectrum of Fig. 3B are illustrated in different patterns in a such manner that these mass spectra are adapted to be clearly distinguishable from each other. In fact, however, in many cases, a mass spectrum corresponding to one strain and a mass spectrum corresponding to another strain have similar patterns, and it is therefore difficult to distinguish these mass spectra from each other.

The target data acquirer 34 acquires mass spectral data (hereinafter referred to as target data) corresponding to the target sample produced by the analyzer 20. The user can instruct the analyzer 20 to provide the target data acquirer 34 with desired target data by operating the operator 15. While the target data acquirer 34 acquires the target data directly from the analyzer 20 in the example of Fig. 2, the present invention is not limited to this. In the case where the target data produced by the analyzer 20 is stored in the storage device 14, the target data acquirer 34 may acquire the target data from the storage device 14.

The discriminator 35 discriminates a strain of the target sample based on the discrimination analysis model produced by the model producer 33 and the target data acquired by the target data acquirer 34. More specifically, the discriminator 35 performs pattern authentication between the mass spectrum based on the target data and each of the discrimination analysis models corresponding to the plurality of strains. A strain that corresponds to a discrimination analysis model that has the highest degree of coincidence with the mass spectrum is discriminated as the strain of the target sample. The discriminator 35 allows the display 16 to display the discriminated strain.

### (3) Strain Discrimination Processing

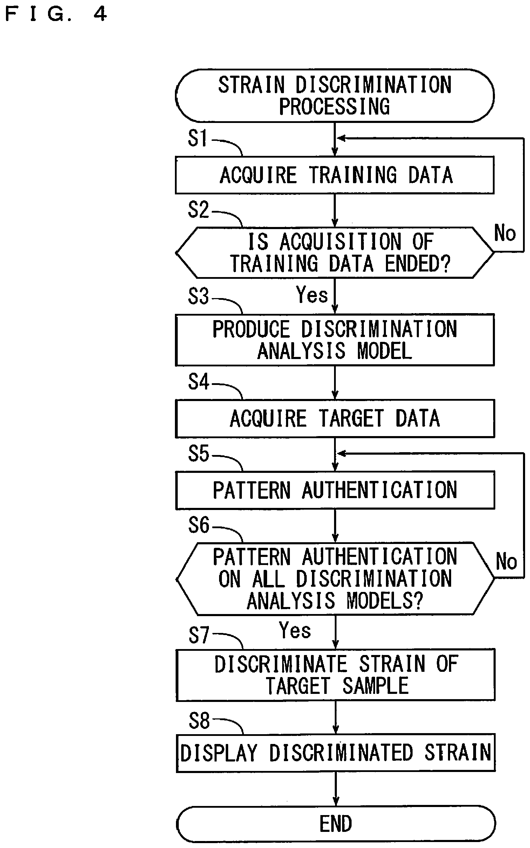

Fig. 4 is a flowchart showing an algorithm of strain discrimination processing performed by a strain discrimination program. The strain discrimination processing will be described below with use of the strain discriminator 30 of Fig. 2 and the flowchart of Fig. 4. While training data and target data are acquired from the analyzer 20 in the following explanation, these data may be acquired from the storage device 14.

First of all, the training data acquirer 31 acquires training data from the analyzer 20 (step S1). In the present embodiment, each training data and strain information corresponding to the training data are registered in the analyzer 20 in such a manner that these data are linked to each other. As such, the strain information acquirer 32 acquires strain information from the analyzer 20 in step S1.

Next, the training data acquirer 31 determines whether an end of acquisition of the training data is instructed (step S2). The user can instruct the training data acquirer 31 to end the acquisition of the training data by operating the operator 15. If the end of the acquisition of the training data has not been instructed, the training data acquirer 31 returns to the step S1. The steps S1 and S2 are repeated until the end of the acquisition of the training data is instructed. Accordingly, the plurality of training data are acquired.

If the end of the acquisition of the training data has been instructed, the model producer 33 produces a discrimination analysis model based on the training data and the strain information acquired in the step S1 (step S3). In the case where a plurality of sets of training data and strain information are acquired for each of the plurality of strains in the step S1, the model producer 33 produces a discrimination analysis model for each strain. The target data acquirer 34 acquires target data from the analyzer 20 (step S4). The step S4 may be executed simultaneously with the step S3 or may be executed at a time point before the step S4.

The discriminator 35 performs pattern authentication between the discrimination analysis models produced in the step S3 and the mass spectrum based on the target data acquired in the step S4 (step S5). After that, the discriminator 35 determines whether the pattern authentication has been performed on all of the discrimination analysis models produced in the step S3 (step S6). If the pattern authentication has not been performed on all of the discrimination analysis models, the discriminator 35 returns to the step S5. The steps S5 and S6 are repeated until the pattern authentication is performed on all of the discrimination analysis models.

If the pattern authentication has been performed on all of the discrimination analysis models, the discriminator 35 discriminates the strain of the target sample based on a result of the authentication in the step S5 (step S7). Finally, the discriminator 35 allows the display 16 to display the strain discriminated in the step S7 (step S8) and ends the strain discrimination processing.

### (4) Effects

In the mass spectrometer 100 according to the present embodiment, each of the plurality of mass spectral data corresponding to the microorganisms, of which strains are known, is acquired as the training data by the training data acquirer 31. The sample corresponding to each training data includes the additive and also the matrix mixed with the sample. The discrimination analysis models for discriminating the strains based on the plurality of training data acquired by the training data acquirer 31 are produced by the model producer 33 by performing the machine learning.

Moreover, the mass spectral data corresponding to the microorganism, of which strain is unknown, is acquired as the target data by the target data acquirer 34. The sample corresponding to the target data includes the additive and also the matrix mixed the sample. The strain of the microorganism corresponding to the target data acquired by the target data acquirer 34 is discriminated by the discriminator 35 based on the discrimination analysis model for each strain produced by the model producer 33 and the acquired target data.

With this configuration, variations in peak intensity of each training data are reduced. As such, it is possible to produce the discrimination analysis model available for the strain discrimination by performing the machine learning on the acquired plurality of training data. In addition, variations in peak intensity of the target data are reduced similarly to each training data. This makes it possible to discriminate with high accuracy the strain of the microorganism corresponding to the target data based on the produced discrimination analysis model and the target data. As a result, the accuracy of discrimination of the strains of the microorganisms is improved.

### (5) Reference Example

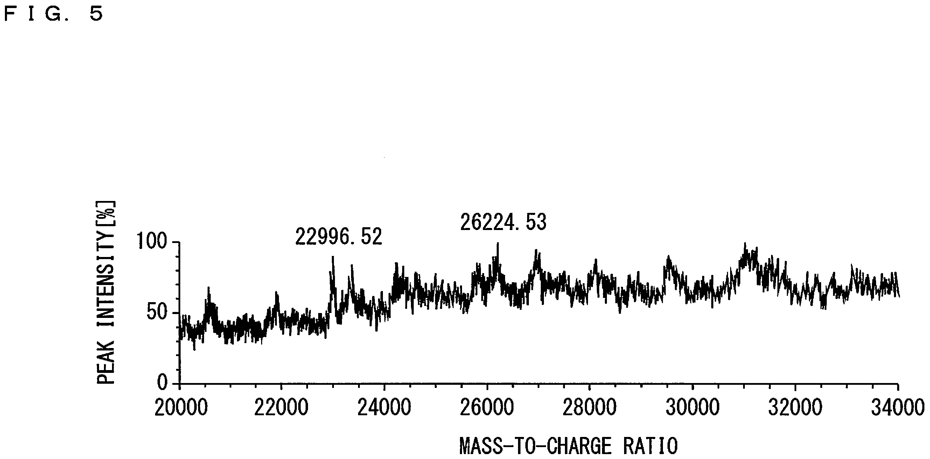

As a technique for discrimination of the strains of the microorganisms, determination of marker peaks is considered as described in the article by Yudai Hotta et al., "Classification of the Genus Bacillus Based on MALDI-TOF MS Analysis of Ribosomal Proteins Coded in S10 and spc Operons," Journal of Agricultural and Food Chemistry, 2011, Vol. 59, No. 10, pp. 5222-5230. Fig. 5 is a diagram showing a mass spectrum of a salmonella. In Fig. 5, the abscissa indicates a mass-to-charge ratio and the ordinate indicates peak intensity. A theoretical mass of a protein expressed only in a strain of the salmonella of Fig. 5 is calculated based on gene information, so that it is presumed that a marker peak is present around the mass-to-charge ratio of 23000.

However, as shown in Fig. 5, a plurality of peaks are present around the mass-to-charge ratio of 23000. Also, each peak intensity is comparatively low. In the case with such lower peak intensities, or in the case where the marker peak is proximate to another peak, it is difficult to stably determine the presence and absence of the marker peak with high accuracy.

As another technique for discrimination of the strains of the microorganisms, main component analysis is considered. More specifically, a plurality of samples of microorganisms classified into any of first to sixth strains were prepared, and a mass spectrum for each sample was measured. Also, a vector composed of a row of peak intensities was produced for each sample, and the main component analysis was performed using the produced plurality of vectors as inputs. An arithmetic operation method for the main component analysis is well known and therefore will not be described herein.

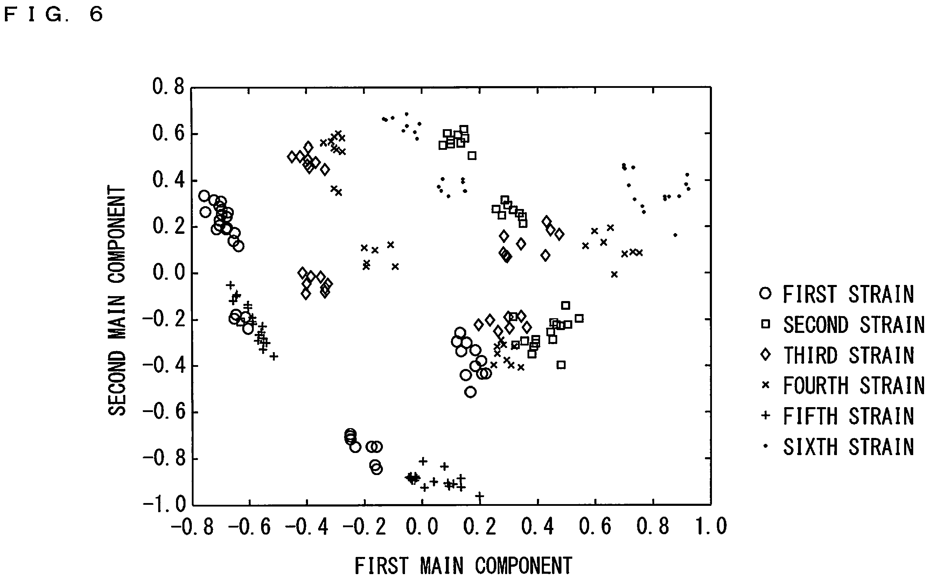

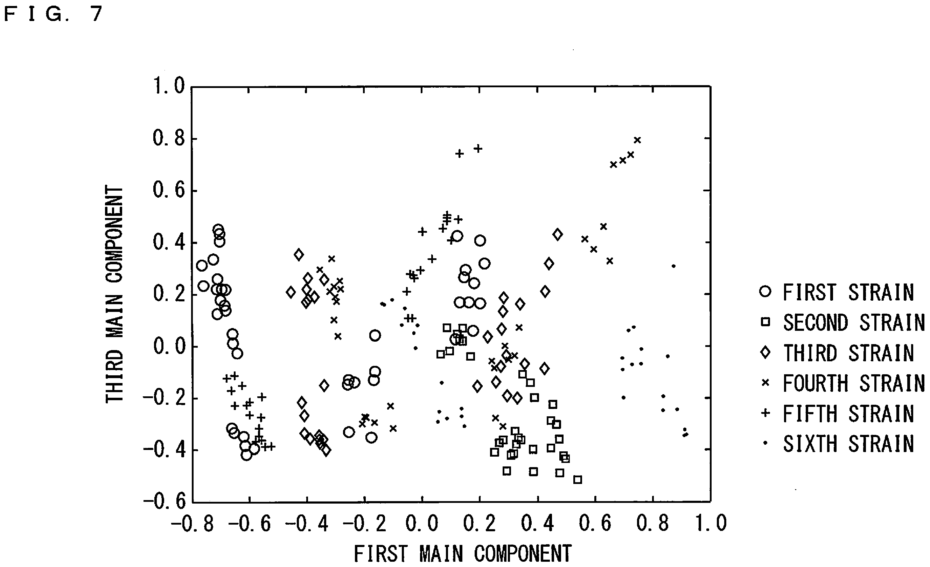

Figs. 6 and 7 are diagrams showing results of the main component analysis with respect to the plurality of samples. In Fig. 6, the abscissa indicates a first main component, and the ordinate indicates a second main component. In Fig. 7, the abscissa indicates the first main component, and the ordinate indicates a third main component. The first to third main components are each represented by a linear combination amount of a plurality of peak intensities. Also, in Figs. 6 and 7, a plurality of indices "○", "□", "◊", "x", "+" and "•" are plotted such that the results of the main component analysis with respect to the samples of the microorganisms classified into the same strain are denoted by the same indices.

As shown in Figs. 6 and 7, the indices corresponding to the same strain tend to form a cluster. However, the clusters formed of the same indices are present separately in a plurality of regions. A cluster formed of one type of indices and a cluster formed of another type of indices overlap with each other. As such, it is suggested that it is difficult to discriminate the strains of the microorganisms with high accuracy by a simple discrimination method such as the main component analysis using the linear combination amount of peak intensities or the like defined as an evaluation function.

### (6) Inventive Example

In an inventive example shown below, the strains of samples were discriminated with use of the discrimination analysis model produced by the SVM based on the aforementioned embodiment. On the other hand, in a comparative example, the strains of samples were discriminated with use of a linear model produced by a general linear discrimination method. An incorrect discrimination rate in each of the inventive example and the comparative example was evaluated by each of holdout validation and cross validation. Details thereof are described below.

### (a) Holdout validation

Mass spectral data with respect to each of 205 samples of which strains are known (hereinafter referred to as simply "data") was produced for two days. More specifically, 107 data were produced on the first day, and 98 data were produced on the second day. A plurality of combinations of training data and target data were defined with use of part or all of the produced 205 data.

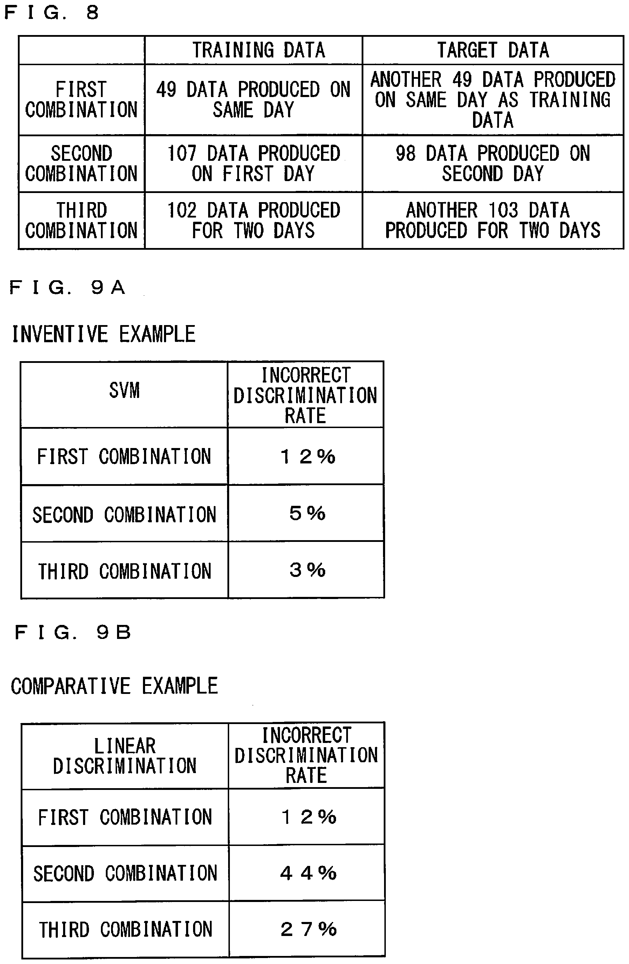

Fig. 8 is a diagram for use in explaining the combinations of training data and target data in holdout validation. As shown in Fig. 8, in a first combination, 49 data produced on the same day were defined as the training data, and another 49 data produced on the same day as the day the training data were produced were defined as the target data. In a second combination, 107 data produced on the first day were defined as the training data, and 98 data produced on the second day were defined as the target data. In a third combination, 102 data produced for two days were defined as the training data, and another 103 data were defined as the target data.

In the inventive example, a strain of each target data in the first combination was discriminated based on the discrimination analysis model produced by the SVM using the training data in the first combination. Similarly, a strain of each target data in the second combination was discriminated based on the discrimination analysis model produced by the SVM using the training data in the second combination. A strain of each target data in the third combination was discriminated based on the discrimination analysis model produced by the SVM using the training data in the third combination.

In the production of the aforementioned 205 data, a matrix was mixed with every sample and an additive was blended in every sample. In the case where no matrix was mixed with the samples or in the case where no additive was blended in the samples, noise components in the data were increased, and variations in peak intensity became larger, and thus, it was impossible to produce a discrimination analysis model.

In the comparative example, a strain of each target data in the first combination was discriminated based on the linear model produced by the linear discrimination method using the training data in the first combination. Similarly, a strain of each target data in the second combination was discriminated based on the linear model produced by the linear discrimination method using the training data in the second combination. A strain of each target data in the third combination was discriminated based on the linear model produced by the linear discrimination method using the training data in the third combination.

Further, the incorrect discrimination rates in the inventive example and the comparative example were evaluated by holdout validation. Figs. 9A and 9B are diagrams showing the incorrect discrimination rates in the inventive example and the comparative example by the holdout validation. As shown in Fig. 9A, the incorrect discrimination rates corresponding to the first to third combinations in the inventive example were 12 %, 5 %, and 3 %, respectively. As shown in Fig. 9B, the incorrect discrimination rates corresponding to the first to third combinations in the comparative example were 12 %, 44 %, and 27 %, respectively. As a result of comparison between the inventive example and the comparative example by the holdout validation, it was confirmed that it was possible to discriminate the strains with high accuracy by using the discrimination analysis model produced by the SVM.

### (b) Cross validation

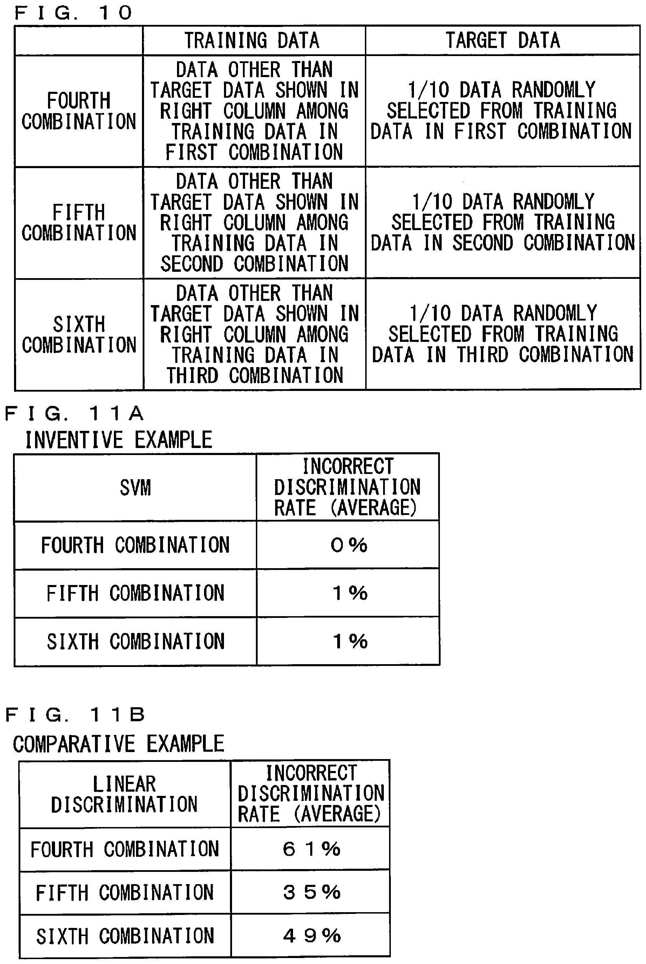

Fig. 10 is a diagram for use in explaining combinations of training data and target data in cross validation. As shown in Fig. 10, in a fourth combination, 1/10 data randomly selected from the training data in the first combination were defined as the target data, and the remaining data were defined as the training data. In a fifth combination, 1/10 data randomly selected from the training data in the second combination were defined as the target data, and the remaining data were defined as the training data. In a sixth combination, 1/10 data randomly selected from the training data in the third combination were defined as the target data, and the remaining data were defined as the training data.

In the cross validation, the random selection of the training data as described above are repeated plural times. Thus, the target data changes and also the training data changes each time the selection is performed.

In the inventive example, each time the training data in the fourth combination was selected, a strain of each target data in the fourth combination was discriminated based on the discrimination analysis model produced by the SVM using the selected training data. Similarly, each time the training data in the fifth combination was selected, a strain of each target data in the fifth combination was discriminated based on the discrimination analysis model produced by the SVM using the selected training data. Each time the training data in the sixth combination was selected, a strain of each target data in the sixth combination was discriminated based on the discrimination analysis model produced by the SVM using the selected training data.

In the comparative example, each time the training data in the fourth combination was selected, a strain of each target data in the fourth combination was discriminated based on the linear model produced by the linear discrimination method using the selected training data. Similarly, each time the training data in the fifth combination was selected, a strain of each target data in the fifth combination was discriminated based on the linear model produced by the linear discrimination method using the selected training data. Each time the training data in the sixth combination was selected, a strain of each target data in the sixth combination was discriminated based on the linear model produced by the linear discrimination method using the selected training data.

Moreover, average incorrect discrimination rates in the inventive example and the comparative example were evaluated by the cross validation. Figs. 11A and 11B are diagrams showing average incorrect discrimination rates in the inventive example and the comparative example by the cross validation. As shown in Fig. 11A, the average incorrect discrimination rates corresponding to the fourth to sixth combinations in the inventive example were 0 %, 1 %, and 1 %, respectively. As shown in Fig. 11B, the average incorrect discrimination rates corresponding to the fourth to sixth combinations in the comparative example were 61 %, 35 %, and 49 %, respectively. As a result of comparison between the inventive example and the comparative example by the cross validation, it was confirmed that it was possible to discriminate the strains with high accuracy by using the discrimination analysis model produced by the SVM.

While preferred embodiments of the present invention have been described above, it is to be understood that variations and modifications will be apparent to those skilled in the art without departing the scope and spirit of the present invention. The scope of the present invention, therefore, is to be determined solely by the following claims.

## Claims

1. A mass spectrometer that discriminates a strain of a microorganism, comprising:
a training data acquirer that acquires, as training data, each of a plurality of mass spectral data with respect to a plurality of samples, each sample including a microorganism of which strain is known, an additive, and a matrix mixed with the sample;
a model producer that produces a discrimination analysis model for discriminating a strain based on the plurality of training data acquired by the training data acquirer by performing machine learning;
a target data acquirer that acquires, as target data, mass spectral data with respect to a sample including a microorganism of which strain is unknown, the additive, and the matrix mixed with the sample; and
a discriminator that discriminates the strain of the microorganism corresponding to the target data acquired by the target data acquirer based on the discrimination analysis model for each strain produced by the model producer and the acquired target data.

2. The mass spectrometer according to claim 1, wherein the additive includes at least one of a compound that inhibits alkali metal-added ion detection and a surfactant.

3. The mass spectrometer according to claim 2, wherein the additive includes a methylenediphosphonic acid or decyl-β-D-maltopyranoside.

4. The mass spectrometer according to any one of claims 1 to 3, wherein the model producer produces the discrimination analysis model by a support vector machine or a neural network.

5. The mass spectrometer according to any one of claims 1 to 4, wherein the matrix includes a sinapic acid.

6. A mass spectrometry method for discriminating a strain of a microorganism, comprising:
acquiring, as training data, each of a plurality of mass spectral data with respect to a plurality of samples, each sample including a microorganism of which strain is known, an additive, and a matrix mixed with the sample;
producing a discrimination analysis model for discriminating a strain based on the acquired plurality of training data by performing machine learning;
acquiring, as target data, mass spectral data with respect to a sample including a microorganism of which strain is unknown, the additive, and the matrix mixed with the sample; and
discriminating the strain of the microorganism corresponding to the acquired target data based on the produced discrimination analysis model for each strain and the acquired target data.

7. The mass spectrometry method according to claim 6, wherein the additive includes at least one of a compound that inhibits alkali metal-added ion detection and a surfactant.

8. The mass spectrometry method according to claim 7, wherein the additive includes a methylenediphosphonic acid or decyl-β-D-maltopyranoside.

9. The mass spectrometry method according to any one of claims 6 to 8, wherein the producing of the discrimination analysis model includes producing the discrimination analysis model by a support vector machine or a neural network.

10. The mass spectrometry method according to any one of claims 6 to 9, wherein the matrix includes a sinapic acid.

11. A mass spectrometry program for discriminating a strain of a microorganism executable by a processor,
the mass spectrometry program causing the processor to execute processes of:
acquiring, as training data, each of a plurality of mass spectral data with respect to a plurality of samples, each sample including a microorganism of which strain is known, an additive, and a matrix mixed with the sample;
producing a discrimination analysis model for discriminating a strain based on the acquired plurality of training data by performing machine learning;
acquiring, as target data, mass spectral data with respect to a sample including a microorganism of which strain is unknown, the additive, and the matrix mixed with the sample; and
discriminating the strain of the microorganism corresponding to the acquired target data based on the produced discrimination analysis model for each strain and the acquired target data.
